# EUROPEAN PATENT SPECIFICATION

(11) **EP 2 740 445 B1**
(45) Date of publication and mention of the grant of the patent: **09.12.2015**
(21) Application number: 12819307.5
(22) Date of filing: 31.07.2012
(51) Int. Cl.: A61F 2/90, A61F 2/94, A61L 31/14, A61L 31/06, A61L 31/08, A61F 2/91

(54) **BIODEGRADABLE STENT WITH GROOVES AND THE PREPARATION METHOD THEREOF**
BIOLOGISCH ABBAUBARER STENT MIT RILLEN UND HERSTELLUNGSVERFAHREN DAFÜR
ENDOPROTHÈSE BIODÉGRADABLE AYANT DES RAINURES ET SON PROCÉDÉ DE PRÉPARATION

(30) Priority: 02.08.2011 CN 201110227994
(43) Date of publication of application: 11.06.2014
(73) Proprietor: Shanghai MicroPort Medical (Group) Co., Ltd., Shanghai 201203 (CN)
(72) Inventor: HUANG, Chubo, Shanghai 201203 (CN); SHI, Xiufeng, Shanghai 201203 (CN); LUO, Qiyi, Shanghai 201203 (CN); TIAN, Hao, Shanghai 201203 (CN); WANG, Yihan, Shanghai 201203 (CN)
(74) Representative: Dennemeyer & Associates S.A.
(86) International application number: PCT/CN2012/079408
(87) International publication number: WO 2013/017069

(56) References cited:
- EP-A2- 1 987 851
- WO-A2-2004/093643
- WO-A2-2008/034050
- CN-A- 101 879 102
- CN-A- 101 938 993
- CN-A- 102 048 602
- CN-A- 102 371 006
- CN-A- 102 379 762
- CN-Y- 2 860 402
- US-A1- 2002 138 154
- US-A1- 2008 071 353
- US-B2- 7 731 740

## Description

### Technical Field

The present invention relates to the medical instrument field. More specifically, the present invention relates to a biodegradable stent with grooves and the preparation method thereof.

### Background

With the gradual progress in the study on biodegradable materials, the performances of such materials are in many aspects suitable for preparing novel biodegradable stents, which are currently under investigation and tests in a significant amount of research institutes. Some clinical data have demonstrated good clinical effects of a biodegradable stent which is getting close to a metallic stent in its physical and delivery performances. However, a biodegradable stent has better biocompatibility and drug carrying property as compared with a metallic stent, and the capability of gradually degrading over time after the treatment; it thus has a great potential and prospect of development (References 1, 6-7 and 9).

Limited by the shape and diameter of coronary arteries, the stent used to treat a coronary stenosis is generally a balloon-expandable stent, which can be pre-compressed onto the balloon of a delivery system, delivered to the site of the coronary stenosis via the delivery system and released by expanding the balloon and retreating the delivery system to the outside of the patient's body. The stent is thereby left in the coronary artery to support and prevent the blood vessel from collapsing. The physicians usually follow a ratio of 1.0-1.1 between the nominal inside diameter of a stent and the inside diameter of the blood vessel to choose a proper stent so as to avoid tear of the vascular endothelium due to overexpansion of the balloon. This would require an expansion/retraction rate of the stent as small as possible after the pressure in the balloon is relieved, so as to avoid additional damages caused by the collapse or displacement of the stent. The expansion/retraction rate of the stent is not only related to the structure but also to the material of the stent. One of the materials useful for preparing biodegradable stents is biodegradable polymer. As compared with metallic materials, the mechanical properties of a polymer, such as yield strength and modulus of elasticity are relatively low. On a stress-strain curve, the strain for a polymer to undergo elastic deformation is in a significantly broader range than a metal. When it comes to the stent, this manifests in that under the same expanding conditions of the balloon, a metallic stent mainly undergoes plastic deformation whereas a biodegradable stent mainly undergoes elastic deformation. The expansion/retraction rate of a biodegradable stent after the pressure in the balloon is relieved is much larger than that of a metallic stent. A high retraction rate of a polymeric stent will definitely cause serious consequences such as collapse or displacement of the stent, and the like. Accordingly, the primary problem to be solved is how to reduce the retraction rate of an expanded polymeric stent.

The Japanese Igaki-Tamai balloon-expandable stent made of polylactic acid takes advantage of the thermoplasticity of the polymer. By filling the balloon with a contrast liquid having a temperature of 80°C, the stent is being heated via thermal conduction during the expansion to a temperature of about 50°C. The temperature is maintained for about 13s, followed by withdrawing the hot contrast liquid and retreating the balloon, leaving the stent in the blood vessel at 37°C to slowly self-expand to its nominal diameter in 20-30min. The expanded stent obtained thereby has a lower expansion/retraction rate and higher radial strength (Reference 2). The disadvantage of this method is that the balloon surfaces uncovered by the stent at either side thereof are put into direct contact with the blood vessel wall at a high temperature (65-70°C for several seconds), causing necrosis of the arterial wall and a subsequent complication of smooth muscle cell proliferation, and eventually, in-stent restenosis.

US7731740 B2 relates to a polymeric stent resistant to relaxation-related negative recoil when implanted in the lumen of a blood vessel. However, this document is silent as to the use of magnetic nanoparticles disposed within the grooves of the stent.

Magnetic nanoparticles have some unique properties. For example, magnetic nanoparticles can convert magnetic energy to thermal energy in an alternating magnetic field (AMF). Hyperthermia is a novel thermal therapy developed on the basis of this property. Research by Jordan indicates that, the site of tumor with absorbed magnetic nanoparticles can reach a temperature of 47°C after being heated with an external AMF for 30min and the cancer cells can be killed without significantly affecting nearby healthy tissues (Reference 3). Hilger et al. injected magnetic particles into cancer and raised its temperature to a high temperature of 58°C in a magnetic field within a short period of time (2-5min), realized the purpose of magnetic targeting and thermal ablation (Reference 4). MagForce Nanotechnologies in German successfully developed a magnetic fluid and corresponding equipment that is already registered and liscenced in EU for treating human glioblastoma, and conducted a clinical trial which demonstrated that this method was effective and safe (References 5 and 8). Upon completion of the therapy, magnetic nanoparticles would be left in human organs, but will not produce any systematic toxicity according to the results from follow-up studies, and will be eliminated from the body through intestine and kidney eventually.

In light of the above, there is a demand in this field for novel biodegradable stent which has a low expansion/retraction rate, and will not causes consequences such as collapse or displacement of the stent. Furthermore, the present invention is intended to search for a method for heating stent, which provides local and rapid heating to a biodegradable polymeric stent. Said method will not only allow the stent to undergo thermal plastic deformation during expansion, but also reduce the thermal damage to the vascular wall to a minimum. Since magnetic nanoparticles generate heat under the impact of an external AMF, this property can be used to realize local heating to the stent.

### Summary of the Invention

To solve the foresaid technical problem, the present invention provides a biodegradable polymeric stent with grooves, characterized in that said stent comprises a stent body, magnetic nanoparticles and a coating layer, wherein the grooves are excavated on the stent body and magnetic nanoparticles are disposed in the grooves; and the stent is heated by using an external alternating magnetic field to increase the temperature of magnetic nanoparticles.

In the present invention, localized or integral heating to the stent is realized by excavating grooves on the stent made of a biodegradable polymeric material, placing magnetic nanoparticles in the grooves, coating the outside of the stent body and causing magnetic nanoparticles to generate heat by increasing the temperature thereof via an external alternating magnetic field. In this way, the stent undergoes thermal plastic deformation during the balloon expansion process and forms a fixed structure after being cooled. Thereby the expansion/retraction rate of the stent is reduced. When the planted stent is gradually degraded, the associated magnetic nanoparticles will be released gradually, absorbed by the human body and excreted and eliminated from the body. This mode of heating is focused, has temperature controllability, and causes less damage to the surrounding environment.

The stent body and the coating layer can be made of the same biodegradable material, or can be made of different biodegradable materials. The biodegradable materials include, but are not limited to, for example, polylactic acid, polyglycolic acid, copolymers of polylactic acid and polyglycolic acid, polycaprolactone, polydioxanone, polyanhydride, tyrosine-derived polycarbonate and the like. Depending on the materials, the degradation period of the stent in the body can be optionally from a few months to three years.

The grooves on the stent body can be arranged at sites of the stent where a deformation is expected, or can be distributed at all sites of the stent. The stent body is 80-200µm wide and 80-200µm thick, and the groove is 30-150µm wide and 30-150µm deep.

The magnetic nanoparticle is a ferromagnetic or superparamagnetic nanoparticle which is capable of generating a large quantity of heat under the impact of an external alternating magnetic field and has desired biocompatibility. The magnetic nanoparticle comprises γ-Fe₂O₃, Fe₃O₄, Ni, Co, Fe, FeCo, NiFe, CoFeO, NiFeO, MnFeO and the like, and a derivative of these particles modified by a small organic molecule, an organic polymer or an inorganic nano material. The material used for modification comprises silane coupling agent, polyethylene glycol, polyvinyl pyrrolidone, polystyrene, polyacrylic acid, polymethyl methacrylate, polyacrylamide and copolymers thereof, polypeptide and block copolypeptide, gelatin, amylopectin, dextran, chitosan and choline phosphate, dopamine, SiO₂ and the like. The magnetic nanoparticle has a size of 10-100 nm.

The procedure to dispose magnetic nanoparticles is described as follows. Magnetic nanoparticles and a solvent are formulated into a magnetic fluid with a certain concentration. The fluid is added to the grooves in drops with a micro-needle. After evaporation of the solvent, magnetic nanoparticles are deposited in the grooves. After several rounds of deposition, the grooves are filled with magnetic nanoparticles. Finally, a coating layer is sprayed onto the outside of the stent body to cover the grooves and other sites of the stent. Alternatively, magnetic nanoparticles, the polymeric material of the stent body or the polymeric material in the coating layer, and a solvent are formulated into a magnetic fluid with a certain concentration. The fluid is added to the grooves in drops with a micro-needle and the grooves are filled up. After evaporation of the solvent, a coating layer is sprayed onto the outside of the stent body to cover the grooves and other sites of the stent.

The thermal radiation range of magnetic nanoparticles is affected by many factors such as the power of the applied magnetic field, the Curie temperature and the distribution density of the particulate material, the heat dissipation capability of heated material and the like. In order to control the heating temperature and heating time of the stent, magnetic nanoparticles having a Curie temperature higher than the glass transition temperature of the material of the stent body are usually selected, and the ratio of magnetic nanoparticles to the stent body is 1:10-1:100 by mass.

The external alternating magnetic field has a frequency of 0-500kHz, and an intensity of 0-30kA/m.

The present invention is realized by the following procedure:
1. Using a biodegradable polymeric material as the material for the stent body to form a tubular product by extrusion and then carving the tubular product with laser engraving techniques to form the stent; excavating grooves on the stent body at sites where a deformation is expected with a laser; disposing magnetic nanoparticles in the grooves; and coating the outside of the stent body.
2. Compressing and holding the above stent on the balloon to deliver the stent to the site of a human coronary artery lesion; turning on the external alternating magnetic field to promote heat generation by magnetic nanoparticles under the effect of magnetic hysteresis, and thereby heating the stent containing the magnetic nanoparticles; removing the external alternating magnetic field away when the temperature of the stent body is increased to be around the glass transition temperature thereof; meanwhile, expanding the stent by balloon to the desired diameter; and then allowing the stent to cool down in the human body to form a fixed structure before releasing pressure and withdrawing the balloon.

In the present invention, magnetic nanoparticles are disposed in grooves on the stent body at sites where a deformation is expected when the biodegradable polymeric stent is expanded, and the outside of the stent body is coated with a layer. When the stent is delivered to the lesion site, the temperature of magnetic nanoparticles in the stent is increased by using an external alternating magnetic field, to a temperature close to the glass transition temperature of the material of the stent body, and thereby, the material of the stent body is heated. When the process to raise the temperature of magnetic nanoparticles is stopped, the balloon is expanded to force the stent to deform plastically under the impact of the heat and the expansionary force from the balloon. Such a plastic deformation is irreversible after the pressure in the balloon is relieved so that the expansion/retraction rate of the stent is greatly reduced. The present invention mainly uses magnetic nanoparticles to heat the stent and will not directly affect the vascular wall.

Magnetic nanoparticles disposed at sites where a deformation is expected when the stent is expanded can selectively heat the sites. In this way, the equivalent weight of magnetic nanoparticles to be used can be reduced, and the heating effect is focused and thus is highly efficient with shorter heating time required. Meanwhile, since the outside of the stent body is covered with a coating layer, hot magnetic nanoparticles will not be in direct contact with the blood or tissues. Thereby, higher safety is achieved.

According to the present invention, the stent is cooled in a human body to form a fixed structure after being heated and expanded. The retraction rate of the expanded stent can be reduced to be lower than 10%, even reaching 1-2%.

### Brief Description of Drawings

To illustrate the technical solutions of the present invention more clearly, the present invention will be introduced briefly below with reference to the figures. Obviously, these figures only represent some specific embodiments of the biodegradable stent of the present application. The structure of the biodegradable stent of the present invention comprises but is not limited to those shown in the figures.
Figure 1 is a general drawing of the stent heating system of the present invention.
Figure 2 shows in details the grooves on the stent rod; Figure 2-a) schematically shows the grooves distributed at sites where a deformation is expected, such as the site of a reinforcement ring; and Figure 2-b) schematically shows the grooves distributed at sites covering all the sites on a stent to be deformed.
Figure 3 shows the cross sectional view of the stent rod.

### Detail Description of the Invention

To help further understand the present invention, the preferred embodiments of the present invention will be described below in conjunction with Examples. This description is only used to exemplify the features and advantages of the present invention, but not to limit the scope of protection of the present invention.

### Example I

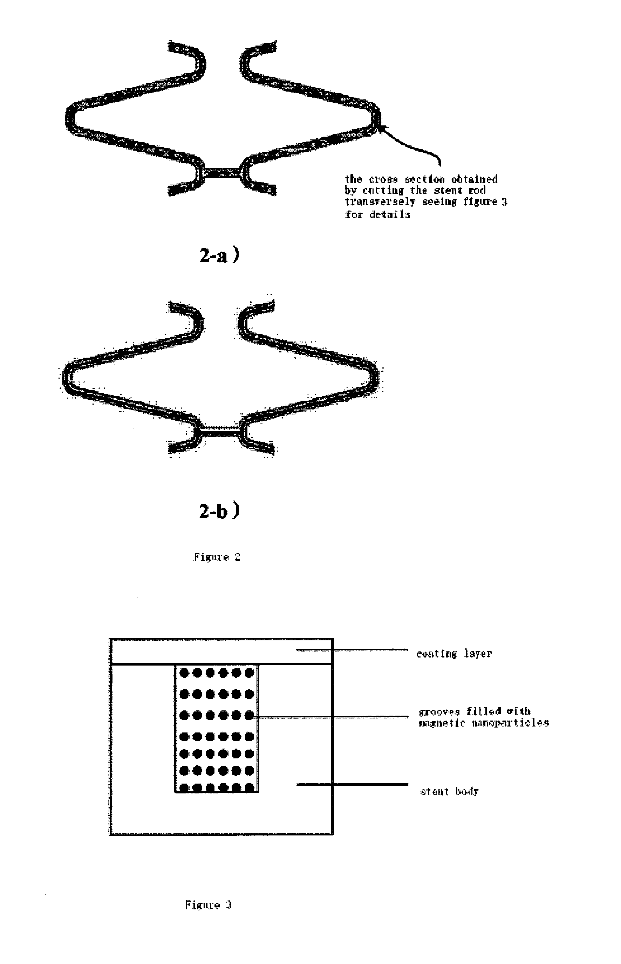

Referring to Figure 2-a), poly(L-lactic acid-co-caprolactone) is extruded to form a tubular product having an external diameter of 2.5mm. The tubular product is processed with laser engraving techniques to form the body of a stent. Grooves that are 150µm deep and 150µm wide are excavated on the stent body at sites of reinforcement rings. Magnetic nanoparticles of γ-Fe₂O₃ having a particle size of 80nm is formulated with dry ethanol as the solvent into a magnetic fluid which is added in drops to the foresaid grooves. The weight of the nanoparticles constitutes 1/20 of the weight of the stent. After complete evaporation of the solvent, a poly-DL-lactic acid (PDLLA) material comprising Rapamycin and derivatives thereof and other similar drugs is sprayed on the outer surface of the stent body to form an external coating layer. The cross section of the stent is shown in Figure 3, representing the structure of the stent. The stent comprises a major body structure, grooves filled with magnetic nanoparticles and an external coating layer, wherein the grooves are located at sites where a deformation is expected.

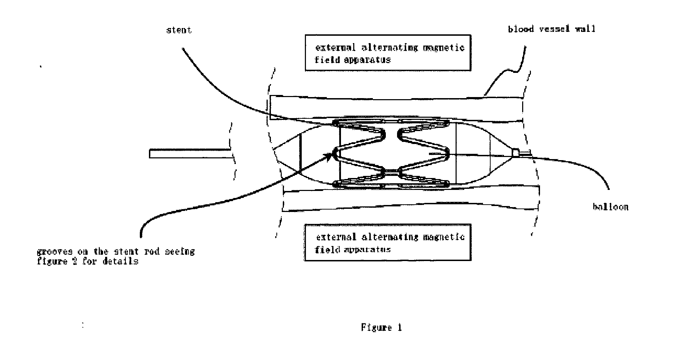

The stent is compressed and held onto a delivery system, and then packed and sterilized. As shown in Figure 1, in a surgery, the stent is delivered to the lesion site by the delivery system. After the position of the external alternating magnetic field is adjusted, the temperature of the stent can be raised to be around 50°C in 3min with a magnetic field having a frequency of 100kHz and an intensity of 18kA/m. Then, the alternating magnetic field is removed away. The stent is expanded by the expansion of the balloon to an external diameter of 3.0mm. The pressure in the balloon is relieved and the delivery system is retreated after the stent is cooled to a temperature below its glass transition temperature. The stent has a final external diameter of 2.76mm and a radial retraction rate of 8%. Three months after the implantation, magnetic nanoparticles are gradually released when the surface coating material PDLLA degrades. Due to their small sizes, magnetic nanoparticles can directly enter into spaces between cells or tissues of the body through blood streams, remain in human organs and will eventually be excreted and eliminated from the body via metabolic pathways.

### Example II

Referring to Figure 2-b), the major body of a stent is produced on the basis of a tubular product having an external diameter of 3.0mm and made of poly-L-lactic acid (PLLA) by a laser cutting machine. Grooves that are 30µm deep and 30µm wide are excavated on the surface of all of the stent rods. Magnetic nanoparticles of MnFeO having a particle size of 10nm and PLLA are dissolved in tetrahydrofuran as the solvent to form a fluid which is sprayed into the grooves of the stent. After complete evaporation of the solvent, the weight of the nanoparticles in the stent constitutes 1/10 of the weight of the stent. A material of poly(L-lactic acid-co-glycolide) (PLAG) comprising Rapamycin is sprayed on the outer surface of the stent body to form an external coating layer. The cross section of the stent is shown in Figure 3, representing the structure of the stent. The stent comprises a major body structure, grooves filled with magnetic nanoparticles and an external coating layer, wherein the grooves are distributed at sites covering all the sites on a stent to be deformed.

The stent is compressed and held onto a delivery system, and then packed and sterilized. As shown in Figure 1, in a surgery, the stent is delivered to the lesion site by the delivery system. After the position of the external alternating magnetic field is adjusted, the temperature of the stent can be raised to be around the glass transition temperature of PLLA, 60°C in 5min with a magnetic field having a frequency of 100kHz and an intensity of 15kA/m. Then, the alternating magnetic field is removed away. The stent is expanded by the expansion of the balloon to an external diameter of 3.5mm. The pressure in the balloon is relieved and the delivery system is retreated after the stent is cooled to a temperature below its glass transition temperature. The stent has a final external diameter of 3.43mm and a radial retraction rate of 2%. Three months after the implantation, magnetic nanoparticles are gradually released when the surface coating material PLAG degrades. Due to their small sizes, magnetic nanoparticles can directly enter into spaces between cells or tissues of the body through blood streams, remain in human organs and will eventually be excreted and eliminated from the body via metabolic pathways.

### Reference List:

1) Antonio Colombo and Evangelia Karvouni, Biodegradable Stents: "Fulfilling the Mission and Stepping Away", Circulation, 2000; 102:371-373.
2) Hideo Tamai, Keiji Igaki, Eisho Kyo, Kunihiko Kosuga, Akiyoshi Kawashima, Initial 1 and 6-Month Results of Biodegradable Poly-L-Lactic Acid Coronary Stents in Humans, Circulation, 2000; 102:399-404.
3) A. Jordan, R. Wust, H. Fdhling, W. John, A. Hinz and R. Felix. Inductive heating of ferrimagnetic particles and magnetic fluids: physical evaluation of their potential of hyperthermia. Int.J.Hyperthermia.1993 (9):51~68.
4) Hilger, et al. Ramans spectroscopy of magnetoliposomes. Investigative Radiology. 2000; 35(3):170~179.
5) Maier-Hauff K, Rothe R, Scholz R et al Intracranial thermotherapy using magnetic nanoparticles combined with external beam radiotherapy: results of a feasibility study on patients with glioblastoma multiforme. J Neurooncol. 2007; 81:53~60.
6) Johannsen M, Gneveckow U, Taymoorian K et al. Morbidity and quality of life during thermotherapy using magnetic nanoparticles in locally recurrent prostate cancer: Results of a prospective phase I trial. Int J Hyperthermia, 2007; 23:315~323.
7) Johannsen M, Gneveckow U, Thiesen B et al. Thermotherapy of prostate cancer using magnetic nanoparticles: feasibility, imaging, and three-dimensional temperature distribution.EurUrol. 2007; 52:1653~1662.
8) Klaus Maier-Hauff, Frank Ulrich, Dirk Nestler, Hendrik Niehoff, Peter Wust, Burghard Thiesen, Helmut Orawa, Volker Budach, Andreas Jordan. Efficacy and safety of intratumoral thermotherapy using magnetic iron-oxide nanoparticles combined with external beam radiotherapy on patients with recurrent glioblastoma multiforme. Journal of Neurooncology, Published online September 16, 2010.
9) Lan Liu et al. Research of pharmacokinetics of Fe2O3-Glu nanoparticles in mice, Journal of Environmental and Occupational Medicine, 2006, 23(1):1~3.

## Claims

1. A biodegradable polymeric stent with grooves, comprising a stent body, magnetic nanoparticles and a coating layer, wherein the grooves are excavated on the stent body and magnetic nanoparticles are disposed in the grooves.

2. The biodegradable polymeric stent according to claim 1, wherein the stent body and the coating layer are made of the same or different biodegradable material selected from polylactic acid, polyglycolic acid, copolymers of polylactic acid and polyglycolic acid, polycaprolactone, polydioxanone, polyanhydride and tyrosine-derived polycarbonate.

3. The biodegradable polymeric stent according to claim 1 or 2, wherein the grooves on the stent body are located at sites where a deformation is expected, or at all sites of the stent.

4. The biodegradable polymeric stent according to any one of the preceding claims, wherein the stent body is 80-200µm wide and 80-200µm thick, and the groove is 30-150µm wide and 30-150µm deep.

5. The biodegradable polymeric stent according to any one of the preceding claims, wherein the magnetic nanoparticles are selected from γ-Fe₂O₃, Fe₃O₄, Ni, Co, Fe, FeCo, NiFe, CoFeO, NiFeO, MnFeO, and derivatives of these particles modified by a small organic molecule, an organic polymer or an inorganic nano material.

6. The biodegradable polymeric stent according to claim 5, wherein a material used for modification is selected from silane coupling agent, polyethylene glycol, polyvinyl pyrrolidone, polystyrene, polyacrylic acid, polymethyl methacrylate, polyacrylamide and copolymers thereof, polypeptide and block copolypeptide, gelatin, amylopectin, dextran, chitosan and choline phosphate, dopamine and SiO₂.

7. The biodegradable polymeric stent according to any one of the preceding claims, wherein the magnetic nanoparticle has a size of 10-100 nm.

8. The biodegradable polymeric stent according to any one of the preceding claims, wherein the ratio of the magnetic nanoparticles to the stent body is 1:10-1:100 by mass.

9. The biodegradable polymeric stent according to any one of the preceding claims, wherein the magnetic nanoparticles are selected to have a Curie temperature higher than the glass transition temperature of the material of the stent body.

10. The biodegradable polymeric stent according to any one of the preceding claims, wherein the temperature of the magnetic nanoparticles is raised by an external alternating magnetic field so as to heat the stent, and the stent is cooled within a human body to form a fixed structure after being heated and expanded, and the retraction rate of the expanded stent is reduced to be lower than 10%.

11. The biodegradable polymeric stent according to claim 10, wherein the external alternating magnetic field has a frequency of 0-500kHz, and an intensity of 0-30kA/m.

12. A method for preparing the biodegradable polymeric stent according to any one of the preceding claims, comprising: using a biodegradable polymeric material as the material for the stent body to form a tubular product by extrusion and then carving the tubular product with laser engraving techniques to form the stent; excavating grooves on the stent body at sites where a deformation is expected or at all sites of the stent, with a laser; disposing magnetic nanoparticles in the grooves; and coating the outside of the stent body.

13. The method according to claim 12, wherein the magnetic nanoparticles are disposed through the following procedures:
magnetic nanoparticles and a solvent are formulated into a magnetic fluid with a certain concentration; the fluid is added to the grooves in drops with a micro-needle; after evaporation of the solvent, magnetic nanoparticles are deposited in the grooves; after several rounds of deposition, the grooves are filled with magnetic nanoparticles; finally, a coating layer is sprayed onto the outside of the stent body to cover the grooves and other sites of the stent; or
magnetic nanoparticles, the polymeric material of the stent body or the polymeric material in the coating layer, and a solvent are formulated into a magnetic fluid with a certain concentration; the fluid is added to the grooves in drops with a micro-needle and the grooves are filled up; after evaporation of the solvent, a coating layer is sprayed onto the outside of the stent body to cover the grooves and other sites of the stent.

## Patentansprüche

1. Biologisch abbaubarer polymerer Stent mit Nuten, der einen Stentkörper, magnetische Nanopartikel und eine Überzugsschicht umfasst, wobei die Nuten auf dem Stentkörper ausgehoben sind und magnetische Nanopartikel in den Nuten abgelagert sind.

2. Biologisch abbaubarer polymerer Stent nach Anspruch 1, wobei der Stentkörper und die Überzugsschicht aus dem gleichen oder aus unterschiedlichem biologisch abbaubaren Material hergestellt sind, das aus Polymilchsäure, Polyglykolsäure, Copolymeren von Polymilchsäure und Polyglykolsäure, Polycaprolakton, Polydioxanon, Polyanhydrid und tyrosinabgeleitetem Polycarbonat ausgewählt ist.

3. Biologisch abbaubarer polymerer Stent nach Anspruch 1 oder 2, wobei die Nuten auf dem Stentkörper an Stellen positioniert sind, an denen eine Verformung zu erwarten ist, oder an allen Stellen des Stents.

4. Biologisch abbaubarer polymerer Stent nach einem der vorstehenden Ansprüche, wobei der Stentkörper 80 bis 200 µm breit und 80 bis 200 µm dick ist und die Nut 30 bis 150 µm breit und 30 bis 150 µm tief ist.

5. Biologisch abbaubarer polymerer Stent nach einem der vorstehenden Ansprüche, wobei die magnetischen Nanopartikel aus γ-Fe₂O₃, Fe₃O₄, Ni, Co, Fe, FeCo, NiFe, CoFeO, NiFeO, MnFeO und Derivaten dieser Partikel, die mit einem kleinen organischen Molekül, einem organischen Polymer oder einem anorganischen Nanomaterial modifiziert sind, ausgewählt sind.

6. Biologisch abbaubarer polymerer Stent nach Anspruch 5, wobei ein zum Modifizieren verwendetes Material aus Silankopplungsmittel, Polyethylenglykol, Polyvinylpyrrolidon, Polystyrol, Polyacrylsäure, Polymethylmethacrylat, Polyacrylamid und Copolymeren davon, Polypeptid und Blockcopolypeptid, Gelatine, Amylopektin, Dextran, Chitosan und Cholinphosphat, Dopamin und SiO₂ ausgewählt ist.

7. Biologisch abbaubarer polymerer Stent nach einem der vorstehenden Ansprüche, wobei der magnetische Nanopartikel eine Größe von 10 bis 100 nm aufweist.

8. Biologisch abbaubarer polymerer Stent nach einem der vorstehenden Ansprüche, wobei das Masseverhältnis der magnetischen Nanopartikel zum Stentkörper 1:10 bis 1:100 ist.

9. Biologisch abbaubarer polymerer Stent nach einem der vorstehenden Ansprüche, wobei die magnetischen Nanopartikel so ausgewählt sind, dass sie eine Curie-Temperatur über der Glasübergangstemperatur des Materials des Stentkörpers aufweisen.

10. Biologisch abbaubarer polymerer Stent nach einem der vorstehenden Ansprüche, wobei die Temperatur der magnetischen Nanopartikel durch ein externes Magnetwechselfeld erhöht wird, um den Stent zu erhitzen, und wobei der Stent innerhalb eines humanen Körpers gekühlt wird, um nach dem Erhitzen und Dehnen eine feste Struktur zu bilden, und wobei die Schrumpfrate des gedehnten Stents auf unter 10 % verringert ist.

11. Biologisch abbaubarer polymerer Stent nach Anspruch 10, wobei das externe Magnetwechselfeld eine Frequenz von 0 bis 500 kHz und eine Intensität von 0 bis 30 kA/m aufweist.

12. Verfahren zum Herstellen eines biologisch abbaubaren polymeren Stents nach einem der vorstehenden Ansprüche, das umfasst: Verwenden eines biologisch abbaubaren polymeren Materials als Material für den Stentkörper, um durch Extrusion ein röhrenförmiges Produkt zu bilden, und danach Schneiden des röhrenförmigen Produkts mit einer Lasergravurtechnik, um den Stent zu bilden; Ausheben von Nuten auf dem Stentkörper an Stellen, an denen eine Verformung zu erwarten ist, oder an allen Stellen des Stents mit einem Laser; Ablagern von magnetischen Nanopartikeln in den Nuten; und Überziehen des Äußeren des Stentkörpers.

13. Verfahren nach Anspruch 12, wobei die magnetischen Nanopartikel mithilfe der folgenden Verfahrensweisen abgelagert werden:
magnetische Nanopartikel und ein Lösungsmittel werden in ein magnetisches Fluid mit einer gewissen Konzentration formuliert; das Fluid wird tropfenweise mit einer Mikronadel zu den Nuten hinzugefügt; nach Verdampfung des Lösungsmittels werden magnetische Nanopartikel in den Nuten abgelagert; nach mehreren Ablagerungsdurchgängen sind die Nuten mit magnetischen Nanopartikeln gefüllt; schließlich wird eine Überzugsschicht auf das Äußere des Stentkörpers gesprüht, um die Nuten und andere Stellen des Stents zu bedecken; oder
magnetische Nanopartikel, das polymere Material des Stentkörpers oder das polymere Material in der Überzugsschicht und ein Lösungsmittel werden in ein magnetisches Fluid mit einer gewissen Konzentration formuliert; das Fluid wird tropfenweise mit einer Mikronadel zu den Nuten hinzugefügt und die Nuten werden aufgefüllt; nach Verdampfung des Lösungsmittels wird eine Überzugsschicht auf das Äußere des Stentkörpers gesprüht, um die Nuten und andere Stellen des Stents zu bedecken.

## Revendications

1. Endoprothèse polymère biodégradable dotée de rainures, comprenant un corps d'endoprothèse, des nanoparticules magnétiques et une couche de revêtement, dans laquelle les rainures sont excavées sur le corps d'endoprothèse et les nanoparticules magnétiques sont disposées dans les rainures.

2. Endoprothèse polymère biodégradable selon la revendication 1, dans laquelle le corps d'endoprothèse et la couche de revêtement sont faits en des matériaux biodégradables identiques ou différents choisis parmi le poly(acide lactique), le poly(acide glycolique), les copolymères de poly(acide lactique) et de poly(acide glycolique), la polycaprolactone, la polydioxanone, le polyanhydride et le polycarbonate dérivé de tyrosine.

3. Endoprothèse polymère biodégradable selon la revendication 1 ou 2, dans laquelle les rainures sur le corps d'endoprothèse sont situées en des sites où une déformation est attendue, ou en tous les sites de l'endoprothèse.

4. Endoprothèse polymère biodégradable selon l'une quelconque des revendications précédentes, dans laquelle le corps d'endoprothèse a une largeur de 80 à 200 µm et une épaisseur de 80 à 200 µm, et la rainure a une largeur de 30 à 150 µm et une profondeur de 30 à 150 µm.

5. Endoprothèse polymère biodégradable selon l'une quelconque des revendications précédentes, dans laquelle les nanoparticules magnétiques sont choisies parmi γ-Fe₂O₃, Fe₃O₄, Ni, Co, Fe, FeCo, NiFe, CoFeO, NiFeO, MnFeO, et les dérivés de ces particules modifiées par une petite molécule organique, un polymère organique ou un nanomatériau inorganique.

6. Endoprothèse polymère biodégradable selon la revendication 5, dans laquelle un matériau utilisé pour la modification est choisi parmi un agent de couplage au silane, le polyéthylèneglycol, la polyvinylpyrrolidone, le polystyrène, le poly(acide acrylique), le poly(méthacrylate de méthyle), le polyacrylamide et ses copolymères, un polypeptide et un copolypeptide séquencé, la gélatine, l'amylopectine, le dextrane, le chitosane et le phosphate de choline, la dopamine et le SiO₂.

7. Endoprothèse polymère biodégradable selon l'une quelconque des revendications précédentes, dans laquelle la nanoparticule magnétique a une taille de 10 à 100 nm.

8. Endoprothèse polymère biodégradable selon l'une quelconque des revendications précédentes, dans laquelle le rapport des nanoparticules magnétiques au corps d'endoprothèse est de 1/10 à 1/100 en masse.

9. Endoprothèse polymère biodégradable selon l'une quelconque des revendications précédentes, dans laquelle les nanoparticules magnétiques sont choisies de façon à avoir une température Curie supérieure à la température de transition vitreuse du matériau du corps d'endoprothèse.

10. Endoprothèse polymère biodégradable selon l'une quelconque des revendications précédentes, dans laquelle la température des nanoparticules magnétiques est augmentée par un champ magnétique alternatif externe de façon que l'endoprothèse soit chauffée, et l'endoprothèse est refroidie à l'intérieur d'un corps humain pour former une structure fixe après avoir été chauffée et expansée, et le taux de rétractation de l'endoprothèse expansée est réduit pour être inférieur à 10 %.

11. Endoprothèse polymère biodégradable selon la revendication 10, dans laquelle le champ magnétique alternatif externe a une fréquence de 0 à 500 kHz, et une intensité de 0 à 30 kA/m.

12. Procédé pour préparer l'endoprothèse polymère biodégradable de l'une quelconque des revendications précédentes, comprenant : l'utilisation d'un matériau polymère biodégradable en tant que matériau pour le corps d'endoprothèse de manière à former un produit tubulaire par extrusion, et ensuite la taille du produit tubulaire par des techniques de gravure au laser de manière à former l'endoprothèse ; l'excavation de rainures sur le corps d'endoprothèse en des sites où une déformation est attendue ou en tous les sites de l'endoprothèse, au moyen d'un laser ; la disposition de nanoparticules magnétiques dans les rainures ; et le revêtement de l'extérieur du corps d'endoprothèse.

13. Procédé selon la revendication 12, dans lequel les nanoparticules magnétiques sont disposées par les procédures suivantes :
des nanoparticules magnétiques et un solvant sont formulés dans un fluide magnétique à une certaine concentration ; le fluide est ajouté aux rainures en gouttes au moyen d'une micro-aiguille ; après évaporation du solvant, les nanoparticules magnétiques sont déposées dans les rainures ; après plusieurs passages de déposition, les rainures sont remplies de nanoparticules magnétiques ; finalement, une couche de revêtement est pulvérisée sur l'extérieur du corps d'endoprothèse de manière à couvrir les rainures et d'autres sites de l'endoprothèse ; ou
des nanoparticules magnétiques, le matériau polymère du corps d'endoprothèse ou le matériau polymère dans la couche de revêtement, et un solvant, sont formulés dans un fluide magnétique à une certaine concentration ; le fluide est ajouté aux rainures en gouttes au moyen d'une micro-aiguille et les rainures sont remplies ; après évaporation du solvant, une couche de revêtement est pulvérisée sur l'extérieur du corps d'endoprothèse de manière à couvrir les rainures et d'autres sites de l'endoprothèse.
